# EUROPEAN PATENT APPLICATION

(11) **EP 2 840 450 A1**
(43) Date of publication of application: **25.02.2015**
(21) Application number: 14185302.8
(22) Date of filing: 07.09.2010
(51) Int. Cl.: G05B 15/00, F25D 29/00, G06Q 10/06, G06Q 50/22, G08B 21/04

(54) **A method and a system for a person's remote assistance**

(30) Priority: 11.09.2009 IT TO20090696
(62) Divisional of application: 10757280.2
(71) Applicant: Indesit Company S.p.A., 60044 Fabriano (AN) (IT)
(72) Inventor: Aisa, Valerio, 60044 Fabriano (AN) (IT); Milani, Monica, 60035 Jesi (AN) (IT); Aiello, Renato, 05016 Ficulle (TR) (IT); Frattesi, Stefano, 60019 Senigallia (AN) (IT)
(74) Representative: Santonicola, Paolo

(57) **Abstract**

The present invention relates to a method and a system for remote assistance wherein a person's behaviours are monitored by interactions with a household appliance.

## Description

The present invention relates to the field of remote home assistance for elderly and/or disabled people, in particular to the possibility of using household appliances as a source of information useful for detecting anomalous behaviours of the assisted persons which may be associated with possible discomfort or danger situations that require the intervention of external qualified and specifically trained personnel.

In brief, the remote assistance systems known in the art schematically consist of three main sub-systems: the set of sensors that generate the information required for ensuring an appropriate monitoring of the assisted person's health and vitality state, the local control unit capable of collecting, storing, processing and transmitting the data continuously generated by the set of sensors, the remote monitoring system that, based on the information constantly sent by the local control system, sends to a suitable assistance structure (e.g. a hospital) connected thereto the information required for ensuring, if necessary, a timely and effective assistance.

Various types of sensors exist which can monitor, directly and in real time, the trend of the assisted person's vital parameters, such as, for example, blood pressure, heart rate, glycemia level, and so on.

Some of these sensors are also available in "wearable" form, i.e. they can be easily associated with the assisted person, thus reducing discomfort to a minimum.

There are also other types of sensors, complementary the above-mentioned ones, which mainly detect information about the assisted person's vitality state and the quality of the environment he/she is living in, e.g. motion sensors, fall sensors, smoke sensors, gas sensors, flood sensors, sensors that monitor water, electricity and gas usage, presence sensor associable with chairs, armchairs, sofas, beds, and door sensors installed in domestic environments, pieces of furniture and household appliances. Among the many patents expressing the "prior art" in this field, one of the most representative ones is the American patent US6002994, which describes in detail the set of sensors which may be associated with a remote home assistance system, making a distinction between sensors that perform direct detections of the assisted person's physiological parameters and sensors that relate to non-physiological parameters, which typically allow to detect events that may be associated with the person's behaviours in the environment where he/she is living. In particular, said patent describes a system for detecting when the door of a household appliance, in particular a refrigerator, is opened and closed, which system can transmit such events to the respective local control system in its turn connected to a remote assistance centre. With reference to the role played by the household appliance as a device for detecting any anomalous behaviours (associable with discomfort or danger situations) of the assisted persons, US6002994 describes how to acquire useful information about the usage of household appliances through suitable external sensors. In the case described therein by way of example, the open or closed state of a refrigerator door is detected through a reed-contact magnetic sensor. The information about the state of the reed contact, which depends on whether the door is open or closed, is constantly acquired by a suitable device capable of transmitting that information to the local control system, e.g. via radio frequency communication.

The method prevalently employed in the prior art for monitoring the frequency of usage of household appliances is therefore based on fitting suitable sensing devices to standard commercial household appliances to detect and transmit to the remote assistance system information about events of interaction with the monitored user. Another known method is based on the use of household devices especially designed for measuring specific physiological parameters of the user when in use. One example of such household devices is described in US4962550, which proposes a water closet capable of automatically analysing the urine of the monitored person.

However, the above known approaches suffer from the drawback that they propose particular solutions which, being non-standard, are not easily marketable and are difficult to implement when, for instance, the household appliance to be monitored is built in a kitchen cabinet.

It is the object of the present invention to overcome the limitations of the prior art. Said object is achieved through a household appliance and a remote assistance method using such a household appliance incorporating the features set out in the annexed claims, which are intended as an integral part of the present description.

The present invention is based on the use of household appliances prearranged for interacting with any remote assistance system for the purpose of contributing to verifying the assisted person's vitality state.

According to the invention, said household appliances belong to the category of network-connectable products, i.e. remotely programmable products fitted with suitable means for connecting to a local area network.

These household appliances are therefore equipped with suitable communication means for connecting to a local area network comprising, in particular, a network interface through which the appliance can communicate with one or more remote assistance systems.

The possibility of remotely programming the household appliances allows each remote assistance system to which they are connected to set the parameters defining how the appliances should collect and transmit the information.

By way of example, a first remote assistance system may require that the appliance send all the data every hour, whereas a second remote assistance system may require that the appliance send only a selection of the collected information, but more frequently.

The presence of a network interface within the household appliance, along with the possibility of programming the latter, allows to monitor interaction events between the appliance and the user without requiring any auxiliary external devices (e.g. consisting, as described in the prior art, of one or more sensors applied externally to the appliance, capable of detecting the user's interaction therewith and of communicating such events to the remote assistance system), thus lacking the technical complexity (and the cost thereof) associated with having to provide the product with additional components, while at the same time causing less discomfort to the user.

Moreover, the household appliance is advantageously equipped with an electronic control system capable of detecting user interaction events by using its own sensing devices, of storing them along with the respective time of occurrence, and of making them available to an external system connected to the same local area network.

Recording the time of occurrence of a certain event is particularly advantageous for remote assistance purposes.

In this field, in fact, recording the time at which interaction between the user and the household appliance takes place is important to identify any changes in the user's behaviour and to make decisions about whether it is necessary or not to intervene locally at the home of the user monitored by the system.

Further objects and advantages of the present invention will become apparent from the following description and from the annexed drawings, which are supplied by way of non-limiting example, wherein:
- Fig. 1 shows a remote assistance system according to the present invention,
- Fig. 2 is a block diagram of a household appliance according to the present invention.

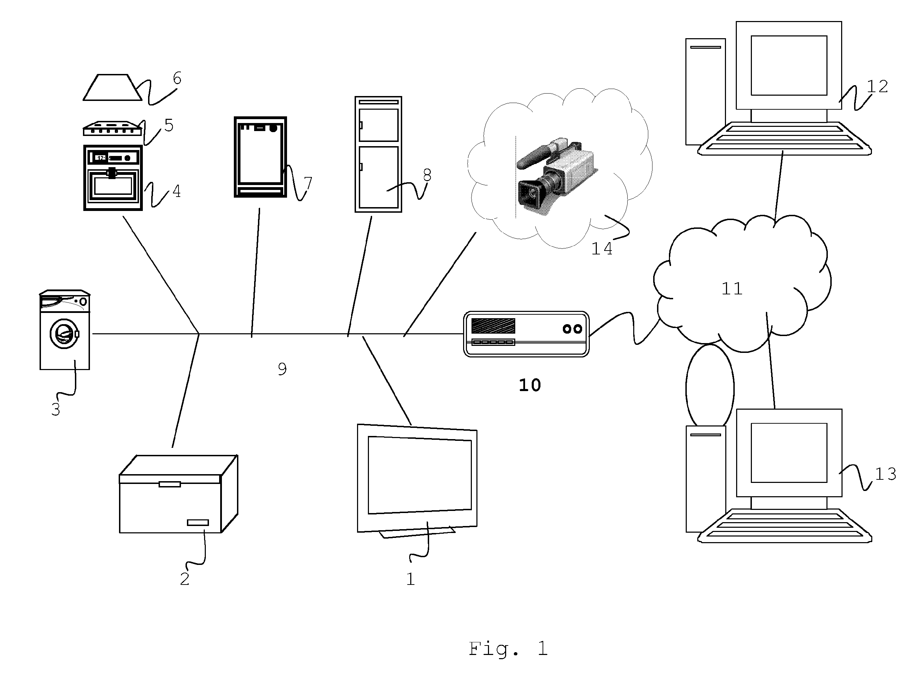

Fig. 1 shows a remote assistance system wherein a plurality of household appliances are connected to a local area network and communicate with a remote assistance centre.

More in detail, a television set 1, a freezer 2, a washing machine 3, an oven 4, a cooking top 5, a hood 6, a dishwasher 7 and a refrigerator 8 are connected to a local area network (LAN) 9 through a suitable interface, which will be further described below.

A control system 10, connected to the same local area network 9, performs the function of collecting data from the household appliances and from any dedicated monitoring devices 14 (i.e. devices specifically conceived for remote assistance purposes, such as video cameras, fall sensors, environmental sensors, devices for detecting physiological parameters, etc.), and also acts as a gateway to an external network 11, in particular the Internet.

The computers 12 and 13 of two remote assistance centres are connected to the same network 11.

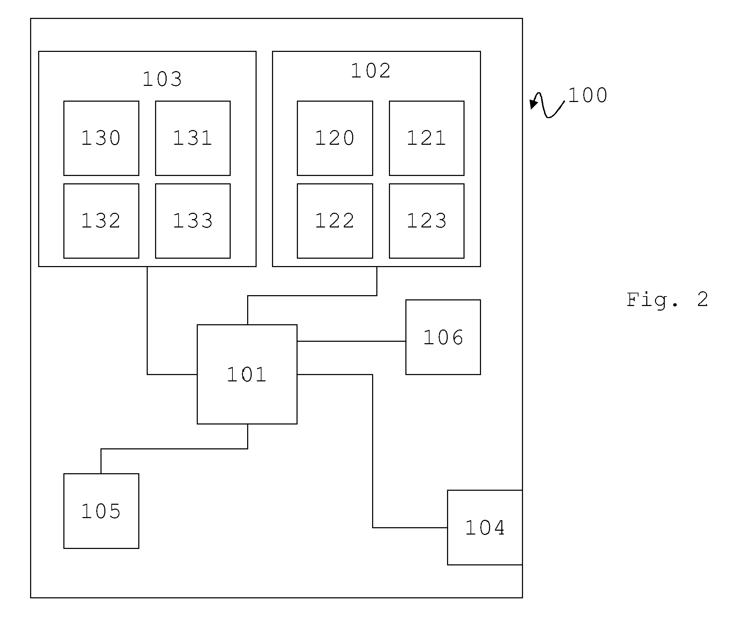

A block diagram of one of the household appliances 1 to 8 is shown in Fig. 2.

The household appliance 100 (whether a television set or a refrigerator or the like) comprises a control unit 101 that controls the operation of the household appliance by acting upon several actuators and components 102.

In the case of a refrigerator, for example, the components 102 controlled by the control unit 101 may include the control panel 120, the compressor 121, an audible alarm 122, the air recirculation fan 123, and so on.

The control unit 101 receives signals from the sensors 103, which detect operating states of the household appliance and any interaction between the user and the household appliance.

Still with reference to the non-limiting example of a refrigerator, the latter may comprise a sensor 130 for detecting when the door is open, a temperature sensor 131, a sensor detecting the current drawn by the compressor 132, a sensor 133 detecting the opening of a certain compartment, e.g. a medicine compartment.

Through the interface 104, the control unit 101 receives and transmits information from/to the network 9, in particular the control unit 101 communicates, through the device 10, with the computers 12 and 13 of the remote assistance centres by sending information detected by the sensors or other information related thereto, as will be described more in detail hereafter.

In the specific case of a refrigerator, which is proposed herein only by way of non-limiting, explanatory example, the recording of the user interaction events is based on monitoring when the doors are opened, which function is carried out by the sensors 130 that monitor the state (open or closed) of the switch typically associated with each refrigerator door.

Every time a door is opened, the refrigerator control unit records that event into a memory (e.g. the memory 105 of Fig. 2) and the time of occurrence thereof, along with the time for which the door has remained open.

To this end, the household appliance (in this case the refrigerator) is preferably equipped with an internal clock 106.

According to the invention, the refrigerator door opening and closing sequences are recorded into a predefined quantity of memory handled as a FIFO (First In - First Out) circular buffer, i.e. such that, when the buffer is full, the newest recorded event automatically eliminates the oldest event so as to free up the necessary space. This ensures that only a limited quantity of memory is used, so as not to jeopardise the proper operation of the product while still being able to constantly store the most recent events.

Preferably, therefore, the memory 105 comprises at least one FIFO-type register, and may possibly comprise a plurality of registers for different events to be monitored.

A statistical analysis of the data going through said circular buffer over time allows the control system to determine, through a suitable algorithm executed by the control unit 101, a profile of the user's habits, thus creating a stable reference base that reflects the user's average behaviour (referred to an observation period of statistically adequate length, managed in «rolling» mode, i.e. susceptible of continuous update) towards the refrigerator at daytime and at nighttime.

By using said reference base concerning the refrigerator usage habits, the refrigerator control unit can, according to the invention, identify any anomalies in the user's behaviour, such as, for example, no doors opened within a time interval longer than the longest one recorded in the profile, or the presence of a very close and unusual sequence of door opening events compared to said profile. Such events do not necessarily indicate a situation of discomfort or danger for the user, but they represent a contribution which, together with other appropriate data acquired by the remote assistance system, may be useful to determine with more certainty when the user actually needs assistance from the hospital structure associated with said remote assistance system.

The control unit 101 comprises code portions which, when executed, allow to process the data received from the sensors and the data contained in the memory area 105 by comparing such data and generating further information representative of anomalous behaviours of the user.

The information thus generated can then be collected into a memory area, e.g. the memory area 105, and/or transmitted through the network interface 104.

In this embodiment, the control unit of the household appliance can autonomously detect an anomalous use of the appliance compared to the user's habits.

As an alternative, the detection of an anomalous use of the appliance may be committed to an external system, e.g. a remote assistance system set up for processing data supplied by the refrigerator.

In other words, the household appliance detects, through sensors, how it is being used by the user, and then sends that information through the network interface 104 to the remote assistance computer 12 or 13, which will in turn re-process it.

The interaction between the household appliance and the remote assistance system may thus take place, according to the invention, in different modes depending on the remote assistance system itself.

The following are a few non-limiting examples of different modes of interaction between the household appliance and the remote assistance system according to the invention:
a) The household appliance autonomously detects any user's anomalous behaviours and signals such events to the remote assistance system when said anomalous behaviours are detected.
b) The household appliance, although capable of detecting any user's anomalous behaviours, limits itself to sending to the remote assistance system, at predefined regular time intervals, the sequence of the events recorded during the last time interval.
c) Upon request from the remote assistance system, the household appliance sends the sequence of the events recorded within the time interval elapsed between the previous request and the current one.
d) The household appliance immediately notifies an interaction event to the remote assistance system when said event takes place.

In order to allow the household appliance to operate in various modes among those listed above, and therefore to communicate with different remote assistance systems, according to the invention the control unit of the household appliance utilises suitable configuration registers which may be set remotely by a computer of a remote assistance system based on the characteristics and requirements of the latter.

In other words, the household appliance according to the present invention behaves towards any remote assistance system as a specific programmable "peripheral", which can provide information about how the user is interacting with it in accordance with the requirements and characteristics of the remote assistance system itself.

The algorithm through which the control unit of the household appliance identifies, according to the invention, anomalous or suspect behavioural situations is based on a comparison between the user's historical profile and current profile, wherein «historical profile» refers to the one derived statistically from the whole past history of the interaction between the user and the household appliance, and «current profile» refers to the one emerging from the most recent history.

In order to better explain the operation of said algorithm according to the invention, a case of a refrigerator will now be taken into consideration wherein, by way of non-limiting example, it will be assumed that the time distribution of the door opening events is catalogued for each day with reference to twenty-four time slots corresponding to the hours.

A door opening event counter is associated with each time slot so that, when such an event occurs in the time slot N (N being comprised between 0 and 23), the respective counter is incremented by one unit.

At the same time, the duration of the door opening event is measured and the value thereof is compared with the current average value in order to verify the extent of any difference and identify possible anomalous situations.

By analysing the hourly distribution of the door opening events with reference to a certain statistically significant time interval, e.g. one week, it is possible to determine any changes with respect to the monthly or annual distribution, i.e. to the user's historical behavioural profile.

By defining appropriate judgement criteria based on said changes, e.g. derived from experimental observations carried out by specialised research institutes, it is possible to discover in a relatively timely manner the onset of any anomalous behaviours of the user.

The information that can be obtained from analysing the hourly distribution of the door opening events and the duration thereof may of course be manifold, depending on the type of algorithm used and on the type of request possibly made by the remote assistance system to which the household appliance is connected, without however departing from the scope of the teachings of the present invention. Some non-limiting examples of such information are: average duration of the door opening events, relative minimum and maximum durations (with reference to a certain time interval), absolute minimum and maximum durations (with reference to the product's history), time frames of most usage of the product, minimum, average and maximum time interval between two successive door opening events, and so on.

It must be stressed that said information may advantageously also be used by the refrigerator control system for optimising its own performance: for example, to identify the time frames of least usage of the product, in which defrosting may take place, if the refrigerator is a no-frost unit with forced ventilation. In fact, in this way defrosting occurs in conditions of utmost efficiency, since no perturbations are caused by the doors being opened, which notoriously make it harder, from an energetic viewpoint, to resume the thermal situation existing in the refrigerator when the defrosting process was activated.

The clock function, which is necessary for defining the time sequence of the user interaction events according to the modes of operation a), b) and c), may, according to the invention, be derived from time to time - when a user interaction event occurs - from the local area network, or it may be managed directly by the control system of the household appliance. In this latter case, according to the invention said control system must be able to update the clock automatically by retrieving this piece of information via the communication network after every power-down, or it must be fitted with an RTC (Real Time Clock) system protected against power failure by a high-capacity battery or capacitor.

The dialogue between the household appliance and the remote assistance system takes place, as aforesaid, through any local area network, which preferably is, without being limited thereto, a radio frequency-based one and may adopt a standard communication protocol such as ZigBee (IEEE-802.15.4) and Z-Wave, characterised by low consumption and by the presence of the meshing function.

The household appliance according to the present invention must, as aforementioned, be fitted with suitable means for communicating directly with a local area network. This can be attained in any one of the following ways:
1) By providing the household appliance, during the production stage, with a suitable communication node (e.g. the interface 104), i.e. an electronic device capable of communicating with the control unit of the product on one side and with an external local area network on the other side.
2) By providing the household appliance with a slot for installing, upon the user's request when the product is purchased or afterwards, a suitable communication node available as an optional accessory.
3) By providing the control unit of the household appliance with a very low-cost technology based on modulating the power absorbed by at least one load of the household appliance, e.g. as described in WO02/21664, whose teachings are intended to be incorporated in the present description, or in the article *"Connecting white goods to a home network at a very low cost",* International Appliance Manufacturing, 2004.

In mode 3), the control unit of the household appliance sends out information by modulating the absorption of one of the appliance's loads controlled by means of a static relay or a triac (a low-cost electronic switch which has now become widespread in the field of household appliances). In this manner, if the mains frequency is 50 or 60 Hertz, the household appliance can transmit 50 or 60 bits per second, respectively. The bits thus transmitted are received by an intelligent electric outlet which acts as a node of the local area network 9. For this purpose, the intelligent outlet is preferably fitted with a transceiver compatible with the ZigBee system, so as to be able to transmit the bits received from the household appliance over the network 9 (or outside of it through the control system 10).

Mode 3) has a limited band, but it offers the advantage that it does not place any additional costs on the product, since it exploits the very same electronics already present in the product itself. This mode may also coexist without any problems with any one of the two preceding modes 1) and 2), which offer better performance at a cost which is however only compatible with high-end products. This means that the mode 3) may, according to the invention, be associated by default with all household appliances equipped with a digital control system, since its cost is virtually zero and its performance, although limited, is nonetheless considered to be absolutely adequate for implementing the remote assistance method described herein. On high-end products requiring a broader band for exchanging information with the outside environment, a communication node will then be installed according to the mode 1) or 2).

Even though for simplicity and clarity the invention has been described herein only with reference to one type of household appliance (a refrigerator) and the user interaction events have only been characterised with reference to monitoring the state of the appliance's doors, the teachings deriving therefrom may also be applied by those skilled in the art to any other type of household appliance and may utilise any other kind of interaction between the user and the household appliance without departing from the protection scope of the present invention as set out in the appended claims. A refrigerator, for example, may comprise one or more compartments, preferably kept at a constant temperature, dedicated to optimally preserving the medicines that the remotely assisted person must take at certain times of the day.

Such compartments are located inside a cooled cell of the refrigerator, and can in turn be closed by a respective door.

In this case, the remote assistance system may, according to the invention, configure the «refrigerator peripheral» in a manner such that it appropriately signals to the user (e.g. by voice synthesis and by lighting up a luminous indicator located on the medicine compartment concerned) the times and modes of intake of said medicines while at the same time notifying the remote assistance system if the user does not follow the recommendations.

To this end, it is conceivable that each medicine compartment is fitted with a suitable sensor detecting when it is being opened by the user.

Should the user not open the compartment to gain access to the medicines, this will be interpreted as a possible dangerous situation for the remotely assisted person.

In a variant embodiment, the control unit of the household appliance is operationally connected to a clock and to said luminous indicator, so that the control unit can change the state of the luminous indicator, in particular by turning it on or changing its colour, at a predefined time.

This predefined time is preferably preset by the user or by a remote device like the computer of the remote assistance system, for the purpose of allowing the door to be opened only at predetermined times when the medicine in question is to be taken.

Advantageously, then, the medicine compartment can be closed with a door which is held in the closed position by a locking mechanism controlled by the control unit.

The locking mechanism is released, thus allowing the door to be opened, when the medicines must be taken.

Referring now to a television set, monitoring power-on times may be useful to detect insomnia situations.

Finally, it is apparent from the above description that the household appliance according to the present invention allows to implement a method for a person's remote assistance, wherein a remote computer (12, 13) detects said person's behaviours thanks to sensors installed in the household appliance itself.

In particular, according to the remote assistance method described herein, the household appliance may, for example, send signals relating to the interaction between a user and said household appliance to a computer (12,13) of a remote assistance centre; said computer (12,13) stores the received data and compares it with the data historically received from the same household appliance, so as to generate anomaly signals as a result of said comparison.

## Claims

1. A method for a person's remote assistance, comprising the steps of
a) detecting interactions between a user and an household appliance (1-8);
b) determining a profile of the user's habits;
c) identifying behavioural anomalies of said person.

2. A method according to claim 1, further comprising between steps a) and b) the step of:
a1) storing said interactions along with the respective time of occurrence.

3. A method according to claim 2, wherein said storing is performed into a predefined quantity of memory handled as a FIFO.

4. A method according to claim 3, **characterized in that** step b) comprises the substep of
b1) performing a statistical analysis of the data going through said circular buffer over time.

5. A method according to any of claims 1 to 4, wherein said interactions comprise the opening of a refrigerator (8) door and/or the opening of a compartment located inside a cooled cell of the refrigerator, and closable by a respective door.

6. A method according to any of the preceding claims, **characterized in that** step c) comprises the substep of
c1) comparing a user's historical profile, derived statistically from the whole past history of the interaction between the user and the household appliance, with current profile referring to the most recent history.

7. A method according to any of the preceding claims wherein said household appliance sends to a remote assistance system, at predefined regular time intervals, the sequence of the events recorded during the last time interval.

8. A method according to any of claims 1 to 6 wherein said household appliance sends, upon request from the remote assistance system, the sequence of the events recorded within the time interval elapsed between the previous request and the current one.

9. A method according to any of claims 1 to 6 wherein said household appliance immediately notifies to the remote assistance system an interaction event when said event takes place.

10. A method according to any of claims 7 to 9 **characterized in that** said household appliance (1-8) communicates with a remote assistance system, according to a mode defined in suitable configuration registers of a control unit (101) of the household appliance, said registers being remotely set by a computer (12,13) of the remote assistance system.

11. A method according to any of preceding claims, **characterized in that** said household appliance (1-8) sends to a computer (12,13) of the remote assistance system signals relating to said interactions between said user and said household appliance, and wherein said computer (12,13) stores the received data and compares it with the data historically received from the same household appliance, so as to generate anomaly signals as a result of said comparison.

12. A method according to any of claims 1 to 6 wherein said household appliance (1-8) autonomously detects any user's anomalous behaviours and signals such events to the remote assistance system when said anomalous behaviours are detected.

13. System for a person's remote assistance, comprising an household appliance (1-8) and a computer of the remote assistance (12,13), said household appliance comprising:
i) at least one sensor adapted to detect an interaction between said household appliance and a person, said person being a user of said household appliance;
ii) an interface for its connection to a communication network (9),
iii) a control unit (101) adapted to communicate with said computer of the remote assistance (12, 13) through said interface (104),
said system being adapted for implementing the method for a person's remote assistance according to any of the preceding claims.

14. System according to claim 13, wherein said control unit (101) is adapted for implementing said step b) and/or c).

15. System according to claim 13, said computer of the remote assistance (12,13) being adapted for implementing said step b) and/or c).
